# EUROPÄISCHE PATENTSCHRIFT

(11) **EP 0 922 442 B1**
(45) Veröffentlichungstag und Bekanntmachung des Hinweises auf die Patenterteilung: **24.08.2005**
(21) Anmeldenummer: 98123394.3
(22) Anmeldetag: 09.12.1998
(51) Int. Cl.: A61F 2/38

(54) **Kniegelenkendoprothese**
Knee joint endoprosthesis
Endoprothèse d'articulation de genou

(30) Priorität: 09.12.1997 DE 19754589
(43) Veröffentlichungstag der Anmeldung: 16.06.1999
(73) Patentinhaber: Engelbrecht, Eckart, Dr., 22359 Hamburg (DE); Hahn, Michael, Dipl.-Ing., 22417 Hamburg (DE)
(72) Erfinder: Engelbrecht, Eckart, Dr., 22359 Hamburg (DE); Hahn, Michael, Dipl.-Ing., 22417 Hamburg (DE)
(74) Vertreter: Heldt, Gert

(56) Entgegenhaltungen:
- US-A- 5 480 443
- US-A- 5 571 196

## Beschreibung

Die Erfindung betrifft eine Kniegelenkendoprothese, bestehend aus einem Femurteil mit Femurgleitlager und einem Tibiateil mit einem Tibiaplateau als Gegenlager.

Viele der heute verwendeten Kniegelenkendoprothesen ersetzen neben dem Hauptgelenk zwischen Ober-und Unterschenkelknochen auch das Gelenk zwischen Kniescheibe und Oberschenkelrolle (Fernoropatellagelenk). Dabei lassen sich heute zwei Möglichkeiten unterscheiden. Bei einem einseitigen Ersatz wird nur das Gleitlager der Oberschenkelrolle (Condylengleitlager) durch ein sogenanntes Patellaschild aus Metall ersetzt, das mit der Femurkomponente verbunden ist. Dabei gleitet die Rückseite der Patella direkt auf den Patellaschild. Da nicht in allen Fällen auf diese Weise eine ausreichende Schmerzbesserung zu erzielen ist, wird statt dieser Lösung häufiger ein totaler Gelenkersatz bevorzugt. Dazu wird zusätzlich die Rückfläche der Patella durch einen entsprechenden Gleitpartner z. B. aus Polyäthylen ersetzt. Zur Verankerung der Patellakomponente ist ein Auffräsen des Knochens durch ein oder mehrere Haftlöcher erforderlich. Der relativ kleine Knochen wird dadurch in seiner Festigkeit deutlich geschwächt. Bei den sehr hohen auftretenden Druck- und Zugbelastungen besteht ein erhöhtes Risiko für eine Patellafraktur sowie für Lockerungen und erhöhten Materialverschleiß der Komponente.

Auf diesem Prinzip, die Patella unmittelbar auf dem Patellaschild der Femurkomponente gleiten zu lassen oder Ober einen mit ihr verbundenen Gleitpartner beispielsweise einem Rückflächenersatz auf dem Patellaschild zu lagern, beruhen auch zahlreiche Lösungen, die aus dem Stand der Technik bekannt geworden sind (US-A-5824105, US-A-5480443). Alle diese Lösungen sind nicht in der Lage, Schmerzfreiheit zu garantieren, da jeweils Relativbewegungen zwischen der Patella und bewegten Implantatteilen stattfinden. Durch diese ist auch eine dauerhafte feste Verankerung des Rücktlächenersatzes der Patella nicht gewährleistet.

Aufgabe der vorliegenden Erfindung ist es daher, diese ernsten und schwer behandelbaren Komplikationen zu vermeiden, ohne dabei die Vorteile des Totalersatzes, d.h. die bessere Schmerzbeseitigung aufzugeben.

Diese Aufgabe wird erfindungsgemäß dadurch gelöst, daß dem Femurgleitlager ventral in einem der Patella zugewandten condylären Bereich eine weder mit der Patella (22) noch mit dem Femurgleitlager verbundene Patellainterpositionsprothese (3) vorgelagert ist.

Grundlage der Idee ist die Tatsache, daß die Patella über die Patellasehne am oberen Schienbein fixiert ist und sich dadurch in Längsrichtung in einer unveränderbaren fest vorgegebenen Position befindet. Das bedeutet, daß nicht die Patella durch Positionsveränderungen über das Condylenlager läuft, sondern das Condylenlager bei Beugebewegungen an der Rückseite der Patella vorbeigleitet. Diese Voraussetzung wird in der Problemlösung ausgenutzt.

Durch die zwischen kondylärem Femurgleitlager und Patellarückseite angeordnete Patellainterpositionsprothese wird vorteilhaft erreicht, daß der eigentliche Gleitvorgang zwischen Patellarückfläche und Femurgleitlager auf den Bereich der dem kondylären Prothesengleitlager zugewandten Rückfläche der Patellainterpositionsprothese (PIP) verlagert wird.

Durch eine nur geringe Rückflächenresektion der Patella wird eine großflächige Auflage der Patellarückfläche auf der Patellainterpositionsprothese angestrebt. Zudem kann konstruktiv ein breitflächiger und damit auch durch kleine Flächenpressungen ausgezeichneter Kontakt zwischen der der Patellarückseite abgewandten Rückseite der Patellainterpositionsprothese und dem kondylären Femurgleitlager bzw. dem Patellaschild hergestellt werden. Auf diese Weise werden die Hauptgleitwege in diesen Bereich verlagert, so daß es zwischen der Patellarückseite und der Patellainterpositionsprothese durch die lockere Auflage nur zu kleinen Relativbewegungen kommt. Auf einen Patellarückflächenersatz z. B. aus Polyäthylen wird bewußt ganz verzichtet. Das Risiko einer aseptischen Lockerung des Patellarückflächenersatzes, vermehrter Materialabrieb sowie ein erhöhtes Risiko einer Patellafraktur wird damit vermieden. Um den konstruktiv vorgegebenen Lauf der jeweiligen Kontaktflächen auf dem Patellaschild einerseits und der PIP andererseits, bzw. zwischen dieser und der Patella zu gewährleisten, ist die Kombination mit einer vollgekoppelten Scharnierendoprothese notwendig.

Gemäß einer bevorzugten Ausführungsform der Erfindung ist die Patellainterpositionsprothese über ein Zwischenstück mit dem Tibiaplateau verbunden. Die Patellainterpositionsprothese ist dabei gegenüber einem ersten Ende des Zwischenstückes um eine Schwenkachse schwenkbar, die etwa parallel zu einer Kniegelenkschwenkachse verläuft. An seinem dem ersten Ende abgewandten zweiten Ende ist das Zwischenstück um eine zur Schwenkachse parallel verlaufende zweite Schwenkachse schwenkbar mit dem Tibiaplateau verbunden. Dadurch wird zwischen Patellainterpositionsprothese und einem benachbarten ventralen Bereich des Tibiaplateaus eine Art Doppelgelenk erreicht, das eine günstige Anlage der Patellainterpositionsprothese in allen Beugegraden an das Femurgleitlager ermöglicht.

Nach einer weiteren bevorzugten Ausführungsform der Erfindung ist die Patellainterpositonsprothese über zwei hintereinander angeordnete Zwischenstücke mit dem Tibiaplateau verbunden, so daß die Patellainterpositionsprothese um drei einander parallele Schwenkachsen gegenüber dem Tibiaplateau verschwenkbar ist.

Dadurch wird die Anlage der Patellainterpositionsprothese gegen das Femurgleitlager noch erhöht.

Gemäß einer weiteren bevorzugten Ausführungsform der Erfindung ist die Patellainterpositonsprothese über zwei in einem Abstand zu einander angeordnete Zwischenstücke mit dem Tibiaplateau verbunden.

Durch die Verwendung von zwei in einem Abstand zueinander angeordneten Zwischenstücken wird eine gewisse Ausrichtung der Patellainterpositionsprothese quer zu den Schwenkachsen ermöglicht, so daß die Anlage der Patellainterpositionsprothese gegen das Femurgleitlager auch quer zu den Schwenkachsen verbessert wird.

Nach einer weiteren bevorzugten Ausführungsform der Erfindung weist die Rückfläche der Patellainterpositionsprothese eine Krümmung auf, die im wesentlichen dem in vertikaler Richtung oberen ventralen Kurvenverlauf des Patellaschildes bzw. des kondylären Femurgleitlagers entspricht. Die Rückfläche der Patellainterpositionsprothese ist durch zwei Auflageflächen an den in vertikaler Richtung unteren abweichenden ventralen Kurvenverlauf des Partellaschildes angepaßt. Die Auflageflächen sind als zwei einander gegenüberliegende parallele Auflageflächen in einem mittleren der Beugeebene benachbarten Bereich des Patellaschildes angeordnet.

Da das Femurgleitlager bereichsweise und die Patellainterpositionsprothese unterschiedliche Krümmungsradien aufweisen, wird durch spezielle Auflageflächen eine optimale Anpassung der Patellainterpositionsprothese an die Krümmung des Patellaschildes erreicht, dessen Krümmungsradius dem ventralen Radius entspricht.

Dadurch kommt es auch im Bereich der stärkeren Beugegrade zu einer optimalen Auflage der Patellainterpositionsprothese, ohne daß bei stärkeren Beugegraden diese um ihre Mittelachse kippt. Diese Stabilität wird dadurch erzielt, daß ihr Radius wesentlich größer ist als der des hinteren Femurgleitlagers.

Weitere Einzelheiten der Erfindung ergeben sich aus der nachfolgenden ausführlichen Beschreibung und den beigefügten Zeichnungen, in denen bevorzugte Ausführungsformen der Erfindung beispielsweise veranschaulicht sind.

In den Zeichnungen zeigen:

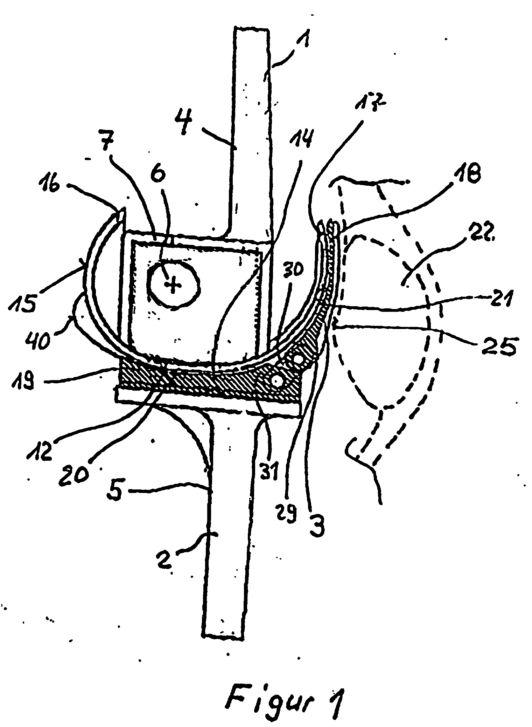

- Figur 1:: Eine Seitenansicht einer Kniegelenkendoprothese mit schraffiert dargestellten Kunststoffteilen und mit gestrichelt angedeuteter Patella,

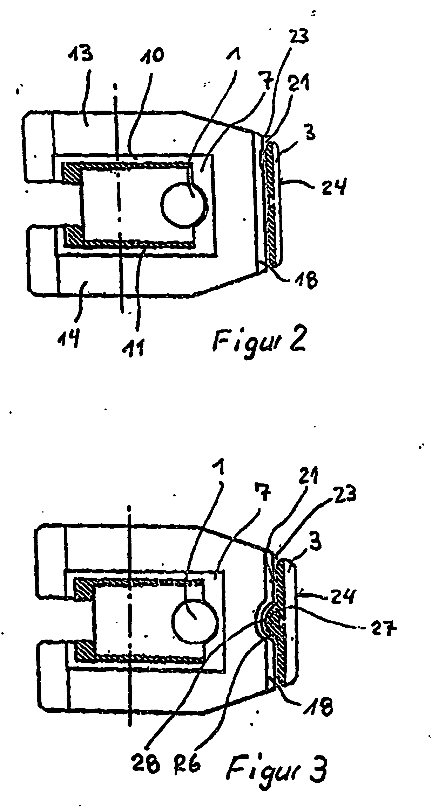

- Figur 2:: eine Draufsicht auf die Kniegelenkendoprothese von Figur 1,
- Figur 3:: eine Draufsicht auf eine Kniegelenkendoprothese entsprechend Figur 2,

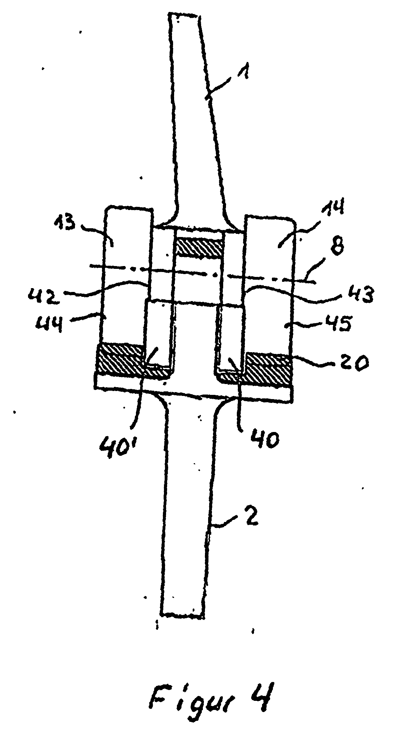

- Figur 4:: eine Rückansicht der Kniegelenkendoprothese von Figur 1,

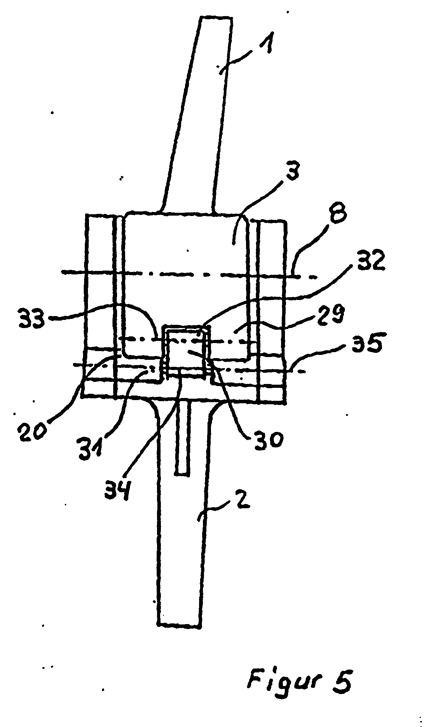

- Figur 5:: eine Vorderansicht der Kniegelenkendoprothese von Figur 1,

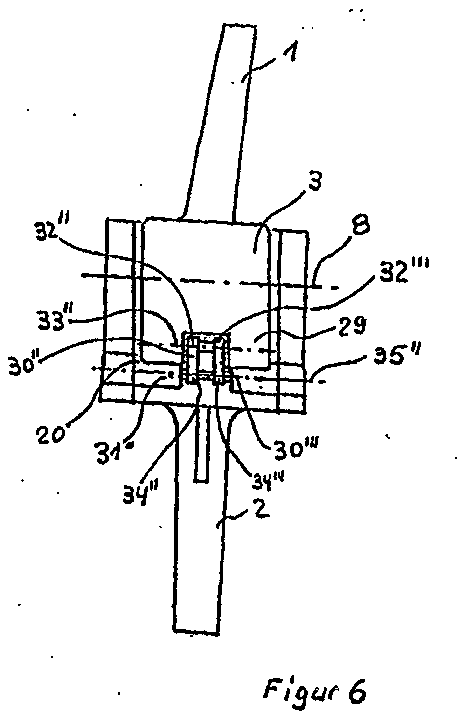

- Figur 6:: eine Vorderansicht einer Kniegelenkendoprothese mit parallelen Zwischenstücken,

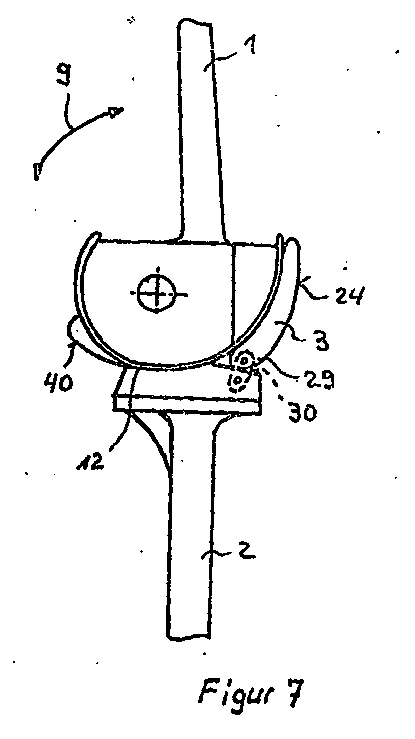

- Figur 7:: eine schematische Seitenansicht einer Kniegelenkendoprothese in gestreckter Stellung,

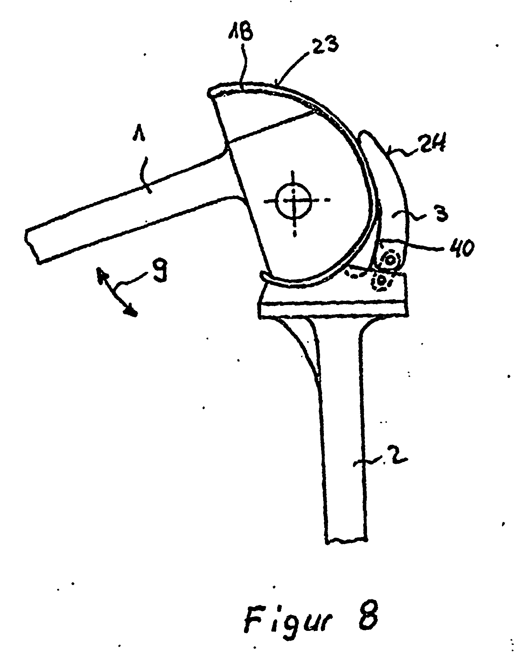

- Figur 8:: eine schematische Seitenansicht der Kniegelenkendoprothese von Fig. 7 in gebeugter Stellung und

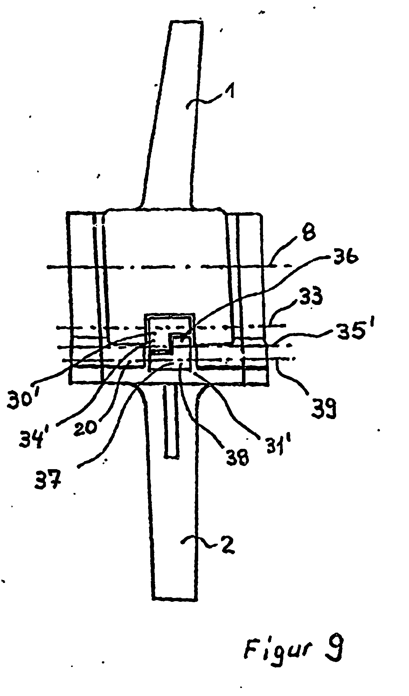

- Figur 9:: eine Vorderansicht einer Kniegelenkendoprothese mit gegeneinander verschwenkbaren Zwischenstücken.

Eine Kniegelenkendoprothese besteht im wesentlichen aus einem Femurteil (1), einem Tibiateil (2) und einer Patellainterpositionsprothese (3). Das Femurteil (1) weist einen Femurschaft (4), das Tibiateil (2) einen Tibiaschaft (5) auf. Die beiden Schäfte (4, 5) weisen in einander abgewandte Richtungen und dienen zur Befestigung der Teile (1, 2) in einem ihnen jeweils benachbarten Knochen. Der Tibiateil (2) kann gegenüber dem Femurteils (1) um eine Welle (6) verschwenkt werden. Die Längsachse der Welle (6) bildet eine Kniegelenkschwenkachse (8), die quer zu einer Beugeebene (9) angeordnet ist, in der die beiden Teile (1, 2) gegeneinander zwischen einer gestreckten und einer gebeugten Stellung verschwenkbar sind.

Im Bereich der dem Tibiateil (2) zugewandten Ausdehnung des Femurteils (1) ist ein Gehäuse (7) angeordnet, das von seitlichen Begrenzungen (10, 11), die sich im wesentlichen planparallel zur Beugeebene (9) erstrecken, seitlich begrenzt wird. An dem dem Tibiateil (2) zugewandten Ende des Gehäuses (7) ist ein Femurgleitlager (12) angeordnet. Das Femurgleitlager (12) besteht aus zwei an den seitlichen Begrenzungen (10, 11) angeordneten Kufen (13, 14), die sich entlang einem Bogen (15) von einem dorsalen Ende (16) in Richtung eines dem dorsalen Ende (16) gegenüberliegenden ventralen Endes (17) erstrecken. Ventral gehen die Kufen (13, 14) in einem kondylärem Bereich über, der als Patellaschild (18) ausgebildet ist. Der Bogen (15) weist vom dorsalen Ende (16) zum ventralen Ende (17) hin verschiedene Krümmungsradien auf. Der Bogen (15) weist im ventralen Bereich des Patellaschildes (18) einen größeren Radius auf als in dem anschließenden Bereich der Kufen (13, 14). An seinem dem Femurgleitlager (12) benachbarten Ende weist das Tibiateil (2) ein Gegenlager (19) auf, das im wesentlichen als ein quer zu dem Tibiaschaft (5) verlaufendes Tibiaplateau (20) ausgebildet ist. Dem Femurgleitlager (12) ist im kondylärem Bereich bzw. dem Patellaschild (18) die Patellainterpositionsprothese (3) vorgelagert. Die Patellainterpositionsprothese (3) liegt mit ihrer dem Patellaschild (18) benachbarten Rückfläche (21) gleitbar an der der Patella (22) zugewandten Lagerfläche (23) des Patellaschildes (18) an. Auf ihrer der Rückfläche (21) abgewandten Seite weist die Patellainterpositionsprothese (3) eine Auflagefläche (24) auf, auf der die Patella (22) mit ihrer der Patellainterpositionsprothese (3) benachbarten Patellarückfläche (25) großflächig lagerbar ist. Durch eine geringe entsprechende Resektion der Patellarückfläche (25) kann diese optimal an die Auflagefläche (24) angepaßt werden.

Nach einer bevorzugten Ausführungsform der Erfindung weist die Patellainterpositionsprothese (3) an ihrer Rückfläche (21) als quer zur Beugeebene (9) angeordnete seitliche Führung (26) eine Erhöhung (27) auf, die in einer entsprechenden Vertiefung (28) des Patellaschildes (18) geführt wird. Die Patellainterpositionsprothese (3) ist an ihrem in vertikaler Richtung unteren Ende (29) über ein Zwischenstück (30) in einem ventralen Bereich (31) des Tibialplateaus (20) angelenkt. Das Zwischenstück (30) weist ein erstes Ende (32) auf, das um eine etwa parallel zur Kniegelenkschwenkachse (8) angeordnete Schwenkachse (33) schwenkbar mit dem unteren Ende (29) der Patellainterpositionsprothese verbunden ist. An seinem dem ersten Ende (32) abgewandten zweiten Ende (34) ist das Zwischenstück (30) um eine parallel zur Schwenkachse (33) angeordnete zweite Schwenkachse (35) schwenkbar mit dem ventralen Bereich (31) des Tibiaplateaus (20) verbunden.

Nach einer anderen Ausführungsform ist das zweite Ende (34') des Zwischenstückes (30') um die zweite Schwenkachse (35') schwenkbar mit einem ersten Ende (36) eines zweiten Zwischenstückes (37) verbunden, das an seinem dem ersten Ende (36) abgewandten zweiten Ende (38) schwenkbar um eine parallel zur zweiten Schwenkachse (35') angeordnete dritte Schwenkachse (39) mit dem ventralen Bereich (31') des Tibiaplateaus (20) verbunden ist.

Nach einer weiteren Ausführungsform der Erfindung ist in einem Abstand zu dem Zwischenstück (30'') ein paralleles Zwischenstück (30''') angeordnet. Die Zwischenstücke (30'', 30''') sind mit ihren ersten Enden (32'', 32''') um die Schwenkachse (33'') schwenkbar mit dem unteren Ende (29) der Patellainterpositionsprothese (3) verbunden. An ihren den ersten Enden (32'', 32''') abgewandten zweiten Enden (34'', 34''') sind die Zwischenstücke (30'', 30''') um eine zweite Schwenkachse (35'') schwenkbar mit dem ventralen Bereich (31'') des Tibiaplateaus (20) verbunden.

Es ist aber grundsätzlich auch möglich, die Patellainterpositionsprothese (3) über ein flexibles Zwischenstück mit dem ventralen Bereich (31) des Tibiaplateaus (20) zu verbinden.

Die Rückfläche (21) der Patellainterpositionsprothese (3) weist eine Krümmung auf, die im wesentlichen dem in vertikaler Richtung oberen ventralen Kurvenverlauf des Patellaschildes (18) entspricht. Die Rückfläche (21) der Patellainterpositionsprothese (3) ist durch zwei zueinander parallel angeordnete Auflageflächen (40, 40'), die am Femurgleitlager (12) angeordnet sind, an den von ventral nach dorsal wegführenden Kurvenverlauf des Patellaschildes (18) bzw. der Kufen (13, 14) angepaßt. Die Auflageflächen (40, 40') sind im mittleren Bereich des Femurgleitlagers (12) angeordnet, und zwar da, wo die Patellainterpositionsprothese (3) auf dem Femurgleitlager (12) aufliegt. Vom mittleren bis zum hinteren dorsalen Bereich der Kufen (13, 14) besteht neben dem Kontakt zwischen der Patellainterpositionsprothese (3) und den beiden einander zugewandten inneren Kanten (42, 43) der Kufen (13, 14) Kontakt außerdem zwischen den äußeren Teilen (44, 45) der Kufen (13, 14) und dem Tibiaplateau (20). Auf diese Weise wird erreicht, daß sich die Kufen auf dem optimalen Radius gegenüber dem Tibiaplateau (20) weiterhin abstützen und trotzdem im inneren Bereich die Patellainterpositionsprothese (3) optimal an den vom oberen Bereich des Patellaschildes (18) vorgegebenen Krümmungsradius angepaßt ist. Dadurch kommt es insbesondere im Bereich der stärkeren Beugegrade zu einer optimalen Auflage der Patellainterpositionsprothese (3), ohne daß bei stärkeren Beugegraden diese um ihre Mittelachse kippt, weil ihr Radius wesentlich größer als der der hinteren dorsalen Kufen (13, 14) in diesem Bereich ist. Die Patellainterpositionsprothese (3) ist aus Metall ausgebildet und auf ihrer dem Femurgleitlager (12) zugewandten Rückfläche (21) mit Kunststoff beschichtet. Als Kunststoff kann beispielsweise Polyäthylen verwendet werden. Es ist auch möglich, die Patellainterpositionsprothese auch auf ihrer Auflagefläche (24) mit Keramik zu beschichten. Grundsätzlich ist es aber auch möglich, die Patellalainterpositionsprothese (3) und auch das Patellaschild (18) komplett aus Keramik auszubilden.

Das Längenverhältnis zwischen dem Zwischenstück (30) und der Patellainterpositionsprothese (3) beträgt beispielsweise 1:5. Die Größe der Patellainterpositionsprothese (3) entspricht etwa der durchschnittlichen Größe eines transversalen Patellaquerschnittes. Die zweite Schwenkachse (35) bzw. die dritte Schwenkachse (39) liegt etwa im vorderen Viertel bzw. vorderen Fünftel des Tibiaplateaus (20). Die Verbindung zwischen Tibiaplateau (20) und dem Zwischenstück (30, 30', 37, 37') ist auswechselbar gestaltet. Daher kann die Patellainterpositionsprothese (3) auch montiert werden, wenn die Kniegelenkendoprothese bereits implantiert ist.

## Patentansprüche

1. Kniegelenkendoprothese bestehend aus einem Femurteil mit Femurgleitlager und einem Tibiateil mit einem Tibiaplateau als Gegenlager, **dadurch gekennzeichnet, daß** dem Femurgleitlager ventral in einem der Patella (22) zugewandten kondylären Bereich eine weder mit der Patella (22) noch mit dem Femurgleitlager verbundene Patellainterpositionsprothese (3) vorgelagert ist.

2. Kniegelenkendoprothese nach Anspruch 1, **dadurch gekennzeichnet, daß** die Patellainterpositionsprothese in einem ventralen Bereich (31) des Tibiaplateaus (20) angelenkt ist.

3. Kniegelenkendoprothese nach Anspruch 1 oder 2, **dadurch gekennzeichnet, daß** die Patellainterpositionsprothese (3) um mindestens eine Schwenkachse (33) schwenkbar ist, die etwa parallel zu einer Kniegelenkschwenkachse (8) verläuft.

4. Kniegelenkendoprothese nach Anspruch 2 oder 3, **dadurch gekennzeichnet, daß** die Patellainterpositionsprothese (3) über mindestens ein Zwischenstück (30) mit dem Tibiaplateau (20) verbunden ist.

5. Kniegelenkendoprothese nach Anspruch 4, **dadurch gekennzeichnet, daß** das Zwischenstück (30) flexibel ausgebildet ist.

6. Kniegelenkendoprothese nach Anspruch 4, **dadurch gekennzeichnet, daß** die Patellainterpositionsprothese (3) gegenüber einem ersten Ende (32) des Zwischenstückes (30) um die Schwenkachse (33) verschwenkbar ist.

7. Kniegelenkendoprothese nach Anspruch 6, **dadurch gekennzeichnet, daß** das Zwischenstück (30) an seinem dem ersten Ende (32) abgewandten zweiten Ende (34) um eine zur Schwenkachse (33) etwa parallel verlaufende zweite Schwenkachse (35) schwenkbar ist.

8. Kniegelenkendoprothese nach Anspruch 7, **dadurch gekennzeichnet, daß** das Zwischenstück (30) mit seinem zweiten Ende (34) mit dem Tibiaplateau (20) verbunden ist.

9. Kniegelenkendoprothese nach Anspruch 7, **dadurch gekennzeichnet, daß** das Zwischenstück (30') über ein zweites Zwischenstück (37) mit dem Tibiaplateau (20) verbunden ist.

10. Kniegelenkendoprothese nach Anspruch 9, **dadurch gekennzeichnet, daß** das zweite Ende (34') des Zwischenstückes (30') mit einem ersten Ende (36) des zweiten Zwischenstückes (37) verbunden ist.

11. Kniegelenkendoprothese nach Anspruch 10, **dadurch gekennzeichnet, daß** das zweite Zwischenstück (37) an einem seinem ersten Ende (36) abgewandten zweiten Ende (38) um eine etwa parallel zur Schwenkachse (33) verlaufende dritte Schwenkachse (39) schwenkbar mit dem Tibiaplateau (20) verbunden ist.

12. Kniegelenkendoprothese nach einem der Ansprüche 4 bis 11, **dadurch gekennzeichnet, daß** die Patellainterpositionsprothese (3) über zwei in einem Abstand zueinander angeordnete Zwischenstücke (30'', 30''') mit dem Tibiaplateau (20) verbunden ist.

13. Kniegelenkendoprothese nach einem der Ansprüche 1 bis 12, **dadurch gekennzeichnet, daß** das Femurgleitlager (12) an seinem der Patella (22) zugewandten ventralen Bereich (31,31',31'') ein Patellaschild (18) aufweist, auf dem die Patellainterpositionsprothese (3) mit einer Rückfläche (21) gleitbar anliegt.

14. Kniegelenkendoprothese nach Anspruch 13, **dadurch gekennzeichnet, daß** die Patellainterpositionsprothese (3) auf ihrer der Rückfläche (21) abgewandten Seite eine Auflagefläche (24) aufweist, auf der die Patella (22) mit ihrer der Patellainterpositionsprothese (3) zugewandten Patellarückfläche (25) großflächig lagerbar ist.

15. Kniegelenkendoprothese nach Anspruch 13 oder 14, **dadurch gekennzeichnet, daß** die Patellainterpositionsprothese (3) an ihrer Rückfläche (21) in Richtung einer quer zur Kniegelenksschwenkachse (8) angeordneten Beugeebene (9) eine seitliche Führung (26) aufweist.

16. Kniegelenkendoprothese nach Anspruch 15, **dadurch gekennzeichnet, daß** die seitliche Führung (26) als etwa mittig angeordnete Erhöhung (27) ausgebildet ist, die in einer entsprechenden Vertiefung (28) des Patellaschildes (18) geführt wird.

17. Kniegelenkendoprothese nach einem der Ansprüche 13 bis 16, **dadurch gekennzeichnet, daß** die Rückfläche (21) der Kniegelenkendoprothese (3) eine Krümmung aufweist, die im wesentlichen dem in vertikaler Richtung oberen ventralen Kurvenverlauf des Patellaschildes (18) entspricht.

18. Kniegelenkendoprothese nach Anspruch 17, **dadurch gekennzeichnet, daß** die Rückfläche (21) der Patellainterpositonsprothese (3) durch mindestens eine Auflagefläche (40,40') an den von ventral nach dorsal wegführenden abweichenden Kurvenverlauf des Femurgleitlagers (12) angepaßt ist.

19. Kniegelenkendoprothese nach Anspruch 18, **dadurch gekennzeichnet, daß** die Auflagefläche (40,40') in einem mittleren der Beugeebene (9) benachbarten Bereich des Femurgleitlagers (12) angeordnet ist.

20. Kniegelenkendoprothese nach Anspruch 18, **dadurch gekennzeichnet, daß** zwei in einem Abstand einander gegenüberliegende parallele Auflageflächen (40, 40') in einem mittleren der Beugeebene (9) benachbarten Bereich des Patellaschildes (18) angeordnet sind.

21. Kniegelenkendoprothese nach einem der Ansprüche 1 bis 20, **dadurch gekennzeichnet, daß** die Patellainterpositionsprothese (3) aus Kunststoff ausgebildet ist.

22. Kniegelenkendoprothese nach einem der Ansprüche 1 bis 20, **dadurch gekennzeichnet, daß** die Patellainterpositionsprothese (3) aus Metall ausgebildet ist.

23. Kniegelenkendoprothese nach Anspruch 22, **dadurch gekennzeichnet, daß** die Patellainterpositionsprothese (3) an ihrer dem Femurgleitlager (12) zugewandten Rückfläche (21) mit Kunststoff beschichtet ist.

24. Kniegelenkendoprothese nach Anspruch 23, **dadurch gekennzeichnet, daß** die Patellainterpositionsprothese (3) an ihrer der Rückfläche (21) abgewandten Auflagefläche (24) mit Keramik beschichtet ist.

25. Kniegelenkendoprothese nach einem der Ansprüche 21 bis 24, **dadurch gekennzeichnet, daß** als Kunststoff Polyäthylen verwendet wird.

26. Kniegelenkendoprothese nach einem der Ansprüche 1 bis 20, **dadurch gekennzeichnet, daß** die Patellainterpositionsprothese (3) aus Keramik ausgebildet ist.

27. Kniegelenkendoprothese nach einem der Ansprüche 1 bis 26, **dadurch gekennzeichnet, daß** das Patellaschild (18) aus Keramik ausgebildet ist.

28. Kniegelenkendoprothese nach Anspruch 27, **dadurch gekennzeichnet, daß** als Keramik Aluminium-Oxyd vorgesehen ist.

## Claims

1. A knee joint endoprosthesis comprising a femur portion with a femur sliding bearing and a tibia portion with a tibia plateau as a counterpart bearing, **characterised in that** mounted ventrally in front of the femur sliding bearing in a condylar region facing towards the patella (22) is a patella interposition prosthesis (3) which is connected neither to the patella (22) nor to the femur sliding bearing.

2. A knee joint endoprosthesis according to claim 1 **characterised in that** the patella interposition prosthesis is pivotally connected in a ventral region (31) of the tibia plateau (20).

3. A knee joint endoprosthesis according to claim 1 or claim 2 **characterised in that** the patella interposition prosthesis (3) is pivotable about at least one pivot axis (33) which extends substantially parallel to a knee joint pivot axis (8).

4. A knee joint endoprosthesis according to claim 2 or claim 3 **characterised in that** the patella interposition prosthesis (3) is connected to the tibia plateau (20) by way of at least one intermediate portion (30).

5. A knee joint endoprosthesis according to claim 4 **characterised in that** the intermediate portion (30) is of a flexible configuration.

6. A knee joint endoprosthesis according to claim 4 **characterised in that** the patella interposition prosthesis (3) is pivotable about the pivot axis (33) with respect to a first end (32) of the intermediate portion (30).

7. A knee joint endoprosthesis according to claim 6 **characterised in that** the intermediate portion (30) is pivotable at its second end (34) which is remote from the first end (32) about a second pivot axis (35) which extends substantially parallel to the pivot axis (33).

8. A knee joint endoprosthesis according to claim 7 **characterised in that** the intermediate portion (30) is connected with its second end (34) to the tibia plateau (20).

9. A knee joint endoprosthesis according to claim 7 **characterised in that** the intermediate portion (30') is connected to the tibia plateau (20) by way of a second intermediate portion (37).

10. A knee joint endoprosthesis according to claim 9 **characterised in that** the second end (34') of the intermediate portion (30') is connected to a first end (36) of the second intermediate portion (37).

11. A knee joint endoprosthesis according to claim 10 **characterised in that** at a second end (38) which is remote from its first end (36) the second intermediate portion (37) is connected to the tibia plateau (20) pivotably about a third pivot axis (39) which extends substantially parallel to the pivot axis (33).

12. A knee joint endoprosthesis according to one of claims 4 to 11 **characterised in that** the patella interposition prosthesis (3) is connected to the tibia plateau (20) by way of two mutually spaced intermediate portions (30", 30"').

13. A knee joint endoprosthesis according to one of claims 1 to 12 **characterised in that** the femur sliding bearing (12) at its ventral region (31, 31', 31") which is towards the patella (22) has a patella plate (18) against which the patella interposition prosthesis (3) slidably bears with a rear surface (21).

14. A knee joint endoprosthesis according to claim 13 **characterised in that** the patella interposition prosthesis (3) on its side which is remote from the rear surface (21) has a contact surface (24) on which the patella (22) can be supported over a large area with its patella rear surface (25) which faces towards the patella interposition prosthesis (3).

15. A knee joint endoprosthesis according to claim 13 or claim 14 **characterised in that** at its rear surface (21) in the direction of a bending plane (9) arranged transversely with respect to the knee joint pivot axis (8) the patella interposition prosthesis (3) has a lateral guide (26).

16. A knee joint endoprosthesis according to claim 15 **characterised in that** the lateral guide (26) is in the form of a substantially centrally arranged raised portion (27) guided in a corresponding recess (28) in the patella plate (18).

17. A knee joint endoprosthesis according to one of claims 13 to 16 **characterised in that** the rear surface (21) of the knee joint endoprosthesis (3) has a curvature which substantially corresponds to the ventral curve configuration, which is the upper one in the vertical direction, of the patella plate (18).

18. A knee joint endoprosthesis according to claim 17 **characterised in that** the rear surface (21) of the patella interposition prosthesis (3) is adapted by at least one contact surface (40, 40') to the deviating curve configuration, which leads away from ventral towards dorsal, of the femur sliding bearing (12).

19. A knee joint endoprosthesis according to claim 18 **characterised in that** the contact surface (40, 40') is arranged in a central region, adjacent to the bending plane (9), of the femur sliding bearing (12).

20. A knee joint endoprosthesis according to claim 18 **characterised in that** two parallel contact surfaces (40, 40') which are arranged at a mutual spacing are disposed in a central region, adjacent to the bending plane (9), of the patella plate (18).

21. A knee joint endoprosthesis according to one of claims 1 to 20 **characterised in that** the patella interposition prosthesis (3) is made of plastic material.

22. A knee joint endoprosthesis according to one of claims 1 to 20 **characterised in that** the patella interposition prosthesis (3) is made of metal.

23. A knee joint endoprosthesis according to claim 22 **characterised in that** at its rear surface (21) which faces towards the femur sliding bearing (12) the patella interposition prosthesis (3) is coated with plastic material.

24. A knee joint endoprosthesis according to claim 23 **characterised in that** at its contact surface (24) which is remote from the rear surface (21) the patella interposition prosthesis (3) is coated with ceramic.

25. A knee joint endoprosthesis according to one of claims 21 to 24 **characterised in that** polyethylene is used as the plastic material.

26. A knee joint endoprosthesis according to one of claims 1 to 20 **characterised in that** the patella interposition prosthesis (3) is made of ceramic.

27. A knee joint endoprosthesis according to one of claims 1 to 26 **characterised in that** the patella plate (18) is made of ceramic.

28. A knee joint endoprosthesis according to claim 27 **characterised in that** aluminium oxide is provided as the ceramic.

## Revendications

1. Endoprothèse d'articulation du genou comprenant une pièce fémorale avec un palier lisse fémoral, et une pièce tibiale avec un plateau tibial en tant qu'élément conjugué, **caractérisée par le fait que** côté ventral, dans une région condylienne tournée vers la patella (22), une prothèse intercalaire de patella (3), qui n'est liée ni à la patella (22), ni au palier lisse fémoral, est disposée devant le palier lisse fémoral.

2. Endoprothèse d'articulation du genou selon la revendication 1, **caractérisée par le fait que** la prothèse intercalaire de patella (3) est articulée dans une région (31) ventrale du plateau tibial (20).

3. Endoprothèse d'articulation du genou selon la revendication 1 ou 2, **caractérisée par le fait que** la prothèse intercalaire de patella (3) peut pivoter autour d'au moins un axe de pivotement (33), qui est sensiblement parallèle à un axe de pivotement (8) de l'articulation du genou.

4. Endoprothèse d'articulation du genou selon la revendication 2 ou 3, **caractérisée par le fait que** la prothèse intercalaire de patella (3) est liée au plateau tibial (20) par au moins une pièce intermédiaire (30).

5. Endoprothèse d'articulation du genou selon la revendication 4, **caractérisée par le fait que** la pièce intermédiaire (30) est flexible.

6. Endoprothèse d'articulation du genou selon la revendication 4, **caractérisée par le fait que** la prothèse intercalaire de patella (3) peut pivoter autour de l'axe de pivotement (33) par rapport à une première extrémité (32) de la pièce intermédiaire (30).

7. Endoprothèse d'articulation du genou selon la revendication 6, **caractérisée par le fait que** la pièce intermédiaire (30), au niveau de sa seconde extrémité (34) éloignée de la première extrémité (32), peut pivoter autour d'un deuxième axe de pivotement (35) qui est sensiblement parallèle à l'axe de pivotement (33).

8. Endoprothèse d'articulation du genou selon la revendication 7, **caractérisée par le fait que** la pièce intermédiaire (30) est liée au plateau tibial (20) par sa seconde extrémité (34).

9. Endoprothèse d'articulation du genou selon la revendication 7, **caractérisée par le fait que** la pièce intermédiaire (30') est liée au plateau tibial (20) par le biais d'une seconde pièce intermédiaire (37).

10. Endoprothèse d'articulation du genou selon la revendication 10, **caractérisée par le fait que** la seconde extrémité (34') de la pièce intermédiaire (30') est liée à une première extrémité (36) de la seconde pièce intermédiaire (37).

11. Endoprothèse d'articulation du genou selon la revendication 10, **caractérisée par le fait que** la seconde pièce intermédiaire (37), au niveau d'une seconde extrémité (38) éloignée de sa première extrémité (36), est liée au plateau tibial (20) avec possibilité de pivotement autour d'un troisième axe de pivotement (39) qui est sensiblement parallèle à l'axe de pivotement (33).

12. Endoprothèse d'articulation du genou selon une des revendications 1 à 11, **caractérisée par le fait que** la prothèse intercalaire de patella (3) est liée au plateau tibial (20) par deux pièces intermédiaires (30", 30"') disposées à distance l'une de l'autre.

13. Endoprothèse d'articulation du genou selon une des revendications 1 à 12, **caractérisée par le fait que** le palier lisse fémoral (12), au niveau de sa région ventrale (31, 31', 31'') tournée vers la patella (22), présente un bouclier patellaire (18) sur le lequel la prothèse intercalaire de patella (3) prend appui avec possibilité de glissement par une surface dorsale (21).

14. Endoprothèse d'articulation du genou selon la revendication 13, **caractérisée par le fait que** la prothèse intercalaire de patella (3) sur sa face éloignée de la surface dorsale (21) présente une surface d'appui (24) sur laquelle la patella (22) repose de manière surfacique avec sa face postérieure (25) tournée vers la prothèse intercalaire de patella (3).

15. Endoprothèse d'articulation du genou selon la revendication 13 ou 14, **caractérisée par le fait que** la prothèse intercalaire de patella (3), sur face postérieure (21), dans la direction d'un plan de flexion (9) orienté transversalement à l'axe de pivotement (8) de l'articulation de genou, présente un guide latéral (26).

16. Endoprothèse d'articulation du genou selon la revendication 15, **caractérisée par le fait que** le guide latéral (26) est conformé en surélévation (27) sensiblement médiane, qui est guidée dans une dépression (28) correspondante aménagée dans le bouclier patellaire (18).

17. Endoprothèse d'articulation du genou selon une des revendications 13 à 16, **caractérisée par le fait que** la face postérieure (21), de l'endoprothèse d'articulation du genou (3) présente une courbure qui correspond essentiellement au profil de courbure supérieur ventral du bouclier patellaire (18) dans la direction verticale.

18. Endoprothèse d'articulation du genou selon la revendication 17, **caractérisée par le fait que** la face postérieure (21) de l'endoprothèse d'articulation du genou (3) est adaptée par au moins une surface d'appui (40, 40') au profil de courbure du palier lisse fémoral (12) menant du côté ventral au côté dorsal.

19. Endoprothèse d'articulation du genou selon la revendication 18, **caractérisée par le fait que** la surface d'appui (40, 40') est disposée dans une région médiane du palier lisse fémoral (12) voisine du plan de flexion (9).

20. Endoprothèse d'articulation du genou selon la revendication 18, **caractérisée par le fait que** deux surfaces d'appui (40, 40') parallèles disposées à distance l'une de l'autre sont prévues dans une zone médiane du bouclier patellaire (18) voisine du plan de flexion (9).

21. Endoprothèse d'articulation du genou selon une des revendications 1 à 20, **caractérisée par le fait que** la prothèse intercalaire de patella (3) est en matière plastique.

22. Endoprothèse d'articulation du genou selon une des revendications 1 à 20, **caractérisée par le fait que** la prothèse intercalaire de patella (3) est en métal.

23. Endoprothèse d'articulation du genou selon la revendication 22, **caractérisée par le fait que** la prothèse intercalaire de patella (3) sur sa face postérieure (21) tournée vers le palier lisse fémoral (12) est revêtue de matière plastique.

24. Endoprothèse d'articulation du genou selon la revendication 23, **caractérisée par le fait que** la prothèse intercalaire de patella (3) sur sa face de portée (24) éloignée de la face postérieure (21) tournée vers le palier lisse fémoral (12) est revêtue de céramique.

25. Endoprothèse d'articulation du genou selon une des revendications 21 à 24, **caractérisée par le fait qu'**on utilise du polyéthylène comme matière plastique.

26. Endoprothèse d'articulation du genou selon une des revendications 1 à 20, **caractérisée par le fait que** la prothèse intercalaire de patella (3) est en céramique.

27. Endoprothèse d'articulation du genou selon une des revendications 1 à 20, **caractérisée par le fait que** le bouclier patellaire (18) est en céramique.

28. Endoprothèse d'articulation du genou selon la revendication 27, **caractérisée par le fait qu'**il est prévu de l'oxyde d'aluminium comme céramique.
